# EUROPEAN PATENT SPECIFICATION

(11) **EP 1 416 965 B1**
(45) Date of publication and mention of the grant of the patent: **19.12.2007**
(21) Application number: 02766022.4
(22) Date of filing: 16.08.2002
(51) Int. Cl.: A61K 39/395, A61K 38/00, G01N 33/53, G01N 33/567, G01N 33/566, C07K 16/00

(54) **ASSAY METHOD FOR ALZHEIMER'S DISEASE**
ASSAYVERFAHREN FÜR ALZHEIMER-KRANKHEIT
PROCEDE DE DOSAGE DESTINE A LA MALADIE D'ALZHEIMER

(30) Priority: 17.08.2001 US 313221 P; 17.08.2001 US 313224 P; 23.10.2001 US 334987 P
(43) Date of publication of application: 12.05.2004
(73) Proprietor: Washington University, St. Louis, MO 63110 (US); ELI LILLY AND COMPANY, Indianapolis, Indiana 46285 (US)
(72) Inventor: HOLTZMAN, David, M., St. Louis, MO 63141 (US); DEMATTOS, Ronald, St. Louis, MO 63108 (US); BALES, Kelly, R., Cloverdale, IN 46120 (US); CUMMINS, David, J., Indianapolis, IN 46256 (US); PAUL, Steven, M., Carmel, IN 46032 (US); JIA, Audrey Yunhua, Union City, California 94587 (US); TSURUSHITA, Naoya, Palo Alto, California 94306 (US); VASQUES, Maximiliano J., Palo Alto, California 94303 (US)
(74) Representative: Chapman, Paul William
(86) International application number: PCT/US2002/026321
(87) International publication number: WO 2003/015617

(56) References cited:
- WO-A-01/62801
- WO-A1-99/27944
- MEHTA PANKAJ D ET AL: "Plasma and cerebrospinal fluid levels of amyloid beta proteins 1-40 and 1-42 in Alzheimer disease" ARCHIVES OF NEUROLOGY, vol. 57, no. 1, January 2000 (2000-01), pages 100-105, XP002343829 ISSN: 0003-9942
- DEMATTOS RONALD B ET AL: "Brain to plasma amyloid-beta efflux: A measure of brain amyloid burden in a mouse model of Alzheimer's disease" SCIENCE (WASHINGTON D C), vol. 295, no. 5563, 22 March 2002 (2002-03-22), pages 2264-2267, XP002343830 ISSN: 0036-8075
- DEMATOS ET AL.: 'Peripheral anti-Abeta antibody alters CNS and plasma Abeta clearance and decreases brain Abeta burden in a mouse model of Alzheimer's disease' PROC. NATL. ACAD. SCI. USA vol. 98, no. 15, 17 July 2001, pages 8850 - 8855, XP002969676
- JANUS ET AL.: 'Abeta peptide immunization reduces behavioral impairment and plaques in a model of Alzheimer's disease' NATURE vol. 408, no. 21, 28 December 2000, pages 979 - 982, XP002969677

### SEQUENCE LISTING

<110> ELI LILLY AND COMPANY and WASHINGTON UNIVERSITY
<120> ASSAY METHOD FOR ALZHEIMER'S DISEASE
<130> 8792/292
<150> 60/334,987
   <151> 2001-10-23
<150> 60/313,221
   <151> 2001-08-17
<150> 60/313,224
   <151> 2001-08-17
<160> 16
<170> Patent In version 3.1
<210> 1
   <211> 16
   <212> PRT
   <213> Mus sp.
<220>
   <221> MISC_FEATURE
   <222> (1) .. (16)
   <223> LIGHT CHAIN CDR1
<400> 1
   Arg Ser Ser Gln Ser Leu Ile Tyr Ser Asp Gly Asn Ala Tyr Leu His
   1 5 10 15
<210> 2
   <211> 7
   <212> PRT
   <213> Mus sp.
<220>
   <221> MISC_FEATURE
   <223> LIGHT CHAIN CDR2
<400> 2
   Lys Val Ser Asn Arg Phe Ser
   1 5
<210> 3
   <211> 9
   <212> PRT
   <213> Mus sp.
<220>
   <221> MISC_FEATURE
   <222> (1) .. (9)
   <223> LIGHT CHAIN CDR3
<400> 3
   Ser Gln Ser Thr His Val Pro Trp Thr
   1 5
<210> 4
   <211> 5
   <212> PRT
   <213> Mus sp.
<220>
   <221> MISC_FEATURE
   <222> (1)..(5)
   <223> HEAVY CHAIN CDR1
<400> 4
   Arg Tyr Ser Met Ser
   1               5
<210> 5
   <211> 17
   <212> PRT
   <213> Mus sp.
<220>
   <221> MISC_FEATURE
   <222> (1)..(17)
   <223> HEAVY CHAIN CDR2
<400> 5
   Gln Ile Asn Ser Val Gly Asn Ser Thr Tyr Tyr Pro Asp Thr Val Lys
   1               5                   10                  15
   Gly
<210> 6
   <211> 3
   <212> PRT
   <213> Mus sp.
<220>
   <221> MISC_FEATURE
   <222> (1)..(3)
   <223> HEAVY CHAIN CDR3
<400> 6
   Gly Asp Tyr
   1
<210> 7
   <211> 113
   <212> PRT
   <213> Artificial Sequence
<220>
   <223> Humanized antibody
   <220>
   <221> MISC_FEATURE
   <222> (1)..(113)
   <223> HUMANIZED ANTIBODY LIGHT CHAIN VARIABLE REGION
<220>
   <221> MISC_FEATURE
   <222> (88)..(88)
   <223> Xaa at position 88 is Val or Leu
<220>
   <221> MISC_FEATURE
   <222> (105)..(105)
   <223> Xaa at position 105 is Gln or Gly
<220>
   <221> MISC_FEATURE
   <222> (108)..(108)
   <223> Xaa at position 108 is Lys or Arg
<220>
   <221> MISC_FEATURE
   <222> (109)..(109)
   <223> Xaa at position 109 is Val or Leu
<220>
   <221> MISC_FEATURE
   <222> (14)..(14)
   <223> Xaa at position 14 is Thr or Ser
<220>
   <221> MISC_FEATURE
   <222> (15) .. (15)
   <223> Xaa at position 15 is Leu or Pro
<220>
   <221> MISC_FEATURE
   <222> (30).. (30)
   <223> Xaa at position 30 is Ile or Val
<220>
   <221> MISC_FEATURE
   <222> (50)..(50)
   <223> Xaa at position 50 is Arg, Gln, or Lys
<220>
   <221> MISC_FEATURE
   <222> (7)..(7)
   <223> Xaa at position 7 is Ser or Thr
<220>
   <221> MISC_FEATURE
   <222> (2) .. (2)
   <223> Xaa at position 2 is Val or Ile
<400> 7
   Asp Xaa Val Met Thr Gln Xaa Pro Leu Ser Leu Pro Val Xaa Xaa Gly
   1               5                   10                  15
   Gln Pro Ala Ser Ile Ser Cys Arg Ser Ser Gln Ser Leu Xaa Tyr Ser
               20                  25                  30
   Asp Gly Asn Ala Tyr Leu His Trp Phe Leu Gln Lys Pro Gly Gln Ser
           35                  40                  45
   Pro Xaa Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Xaa Gly Val Tyr Tyr Cys Ser Gln Ser
                   85                  90                  95
   Thr His Val Pro Trp Thr Phe Gly Xaa Gly Thr Xaa Xaa Glu Ile Lys
               100                 105                 110
   Arg
<210> 8
   <211> 112
   <212> PRT
   <213> Artificial Sequence
<220>
   <223> Humanized antibody
   <220>
   <221> MISC_FEATURE
   <222> (1) .. (112)
   <223> HUMANIZED ANTIBODY HEAVY CHAIN VARIABLE REGION
<220>
   <221> MISC_FEATURE
   <222> (76) .. (76)
   <223> Xaa at position 76 is Lys or Arg
<220>
   <221> MISC_FEATURE
   <222> (89) .. (89)
   <223> Xaa at position 89 is Glu or Asp
<220>
   <221> MISC_FEATURE
   <222> (107)..(107)
   <223> Xaa at position 107 is Leu or Thr
<220>
   <221> MISC_FEATURE
   <222> (1) .. (1)
   <223> Xaa at position 1 is Glu or GIn
<220>
   <221> MISC_FEATURE
   <222> (7)..(7)
   <223> Xaa at position 7 is Ser or Leu
<220>
   <221> MISC_FEATURE
   <222> (46) .. (46)
   <223> Xaa at position 46 is Glu, Val, Asp, or Ser
<220>
   <221> MISC_FEATURE
   <222> (63)..(63)
   <223> Xaa at position 63 is Thr or Ser
<220>
   <221> MISC_FEATURE
   <222> (75) .. (75)
   <223> Xaa at position 75 is Ala, Ser, Val, or Thr
<400> 8
   Xaa Val Gln Leu Val Glu Xaa Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Xaa Leu Val
           35                  40                  45
   Ala Gln Ile Asn Ser Val Gly Asn Ser Thr Tyr Tyr Pro Asp Xaa Val
       50                  55                  60
   Lys Gly Arg Phe Thr Ile Ser Arg Asp Asn Xaa Xaa Asn Thr Leu Tyr
   65                  70                  75                  80
   Leu Gln Met Asn Ser Leu Arg Ala Xaa Asp Thr Ala Val Tyr Tyr Cys
                   85                  90                  95
   Ala Ser Gly Asp Tyr Trp Gly Gln Gly Thr Xaa Val Thr Val Ser Ser
               100                 105                 110
<210> 9
   <211> 113
   <212> PRT
   <213> Artificial Sequence
<220>
   <223> Humanized antibody
   <220>
   <221> MISC_FEATURE
   <222> (1) .. (113)
   <223> HUMANIZED ANTIBODY LIGHT CHAIN VARIABLE REGION
<400> 9
   Asp Val Val Met Thr Gln Ser Pro Leu Ser Leu Pro Val Thr Leu Gly
   1               5                   10                  15
   Gln Pro Ala Ser Ile Ser Cys Arg Ser Ser Gln Ser Leu Ile Tyr Ser
               20                  25                  30
   Asp Gly Asn Ala Tyr Leu His Trp Phe Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Ser
                   85                  90                  95
   Thr His Val Pro Trp Thr Phe Gly Gln Gly Thr Lys Val Glu Ile Lys
               100                 105                 110
   Arg
<210> 10
   <211> 112
   <212> PRT
   <213> Artificial Sequence
<220>
   <223> Humanized antibody
   <220>
   <221> MISC_FEATURE
   <222> (1)..(112)
   <223> HUMANIZED ANTIBODY HEAVY CHAIN VARIABLE REGION
<400> 10
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Glu Leu Val
           35                  40                  45
   Ala Gln Ile Asn Ser Val Gly Asn Ser Thr Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
               100                 105                 110
<210> 11
   <211> 219
   <212> PRT
   <213> Artificial Sequence
<220>
   <223> Humanized antibody
   <220>
   <221> MISC_FEATURE
   <222> (1) .. (219)
   <223> HUMANIZED ANTIBODY LIGHT CHAIN
<400> 11
   Asp Val Val Met Thr Gln Ser Pro Leu Ser Leu Pro Val Thr Leu Gly
   1               5                   10                  15
   Gln Pro Ala Ser Ile Ser Cys Arg Ser Ser Gln Ser Leu Ile Tyr Ser
               20                  25                  30
   Asp Gly Asn Ala Tyr Leu His Trp Phe Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Ser
                   85                  90                  95
   Thr His Val Pro Trp Thr Phe Gly Gln Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 12
   <211> 442
   <212> PRT
   <213> Artificial Sequence
<220>
   <223> Humanized antibody
   <220>
   <221> MISC_FEATURE
   <222> (1)..(442)
   <223> HUMANIZED ANTIBODY HEAVY CHAIN
<400> 12
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Glu Leu Val
           35                  40                  45
   Ala Gln Ile Asn Ser Val Gly Asn Ser Thr Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
           100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Leu Leu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
   290 295 300
   His Gln Asp Trp Leu Asn Gly Lys Glu Tyr Lys Cys Lys Val Ser Asn
   305 310 315 320
   Lys Ala Leu Pro Ala Pro Ile Glu Lys Thr Ile Ser Lys Ala Lys Gly
   325 330 335
   Gln Pro Arg Glu Pro Gln Val Tyr Thr Leu Pro Pro Ser Arg Asp Glu
   340 345 350
   Leu Thr Lys Asn Gln Val Ser Leu Thr Cys Leu Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Tyr Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 13
   <211> 17
   <212> PRT
   <213> Mouse Variant
<220>
   <221> MISC_FEATURE
   <222> (1) .. (17)
   <223> HEAVY CHAIN CDR2
<220>
   <221> MISC_FEATURE
   <222> (7)..(7)
   <223> Xaa at position 7 is any amino acid, provided that is Xaa at position 8 is
   neither Asp nor Pro and Xaa at position 9 is Ser or Thr, then Xaa at
   position 7 is not Asn
<220>
   <221> MISC_FEATURE
   <222> (8)..(8)
   <223> Xaa at position 8 is any amino acid, provided that Xaa at position 7 is
   Asn and Xaa at position 9 is Ser or Thr, then Xaa at position 8 is Asp or
   Pro
<220>
   <221> MISC_FEATURE
   <222> (9)..(9)
   <223> Xaa at position 9 is any amino acid, provided that Xaa at position 7 is
   Asn and Xaa at position 8 is neither Asp nor Pro, then Xaa at position 9
   is neither Ser nor Thr
<400> 13
   Gln Ile Asn Ser Val Gly Xaa Xaa Xaa Tyr Tyr Pro Asp Thr Val Lys
   1               5                   10                  15
   Gly
<210> 14
   <211> 112
   <212> PRT
   <213> Artificial Sequence
<220>
   <223> Humanized Antibody
<220>
   <221> MISC_FEATURE
   <222> (1) .. (112)
   <223> Deglycosylated Humanized Antibody Heavy Chain Variable Region
<220>
   <221> MISC_FEATURE
   <222> (63)..(63)
   <223> Xaa at position 63 is Thr or Ser
<220>
   <221> MISC_FEATURE
   <222> (1)..(1)
   <223> Xaa at position 1 is Glu or Gln
<220>
   <221> MISC_FEATURE
   <222> (7) .. (7)
   <223> Xaa at position 7 is Ser or Leu
<220>
   <221> MISC_FEATURE
   <222> (46) .. (46)
   <223> Xaa at position 46 is Glu, Val, Asp, or Ser
<220>
   <221> MISC_FEATURE
   <222> (56) .. (56)
   <223> Xaa at position 56 is any amino acid, provided that if Xaa at pos
   ition 57 is neither Asp nor Pro and Xaa at position 59 is S er or
   Thr, then Xaa at position 56 is not Asn
<220>
   <221> MISC_FEATURE
   <222> (57)..(57)
   <223> Xaa at position 57 is any amino acid, provided that if Xaa at pos
   ition 56 is Asn and Xaa at position 58 is Ser or Thr, then Xaa at
   position 57 is Asp or Pro
<220>
   <221> MISC_FEATURE
   '<222> (58)..(58)
   <223> Xaa at position 58 is any amino acid, provided that if Xaa at pos
   ition 56 is Asn and Xaa at position 57 is neither Asp nor P ro, th
   en Xaa at position 58 is neither Ser nor Thr
<220>
   <221> MISC_FEATURE
   <222> (75) .. (75)
   <223> Xaa at position 75 is Ala, Ser, Val, or Thr
<220>
   <221> MISC_FEATURE
   <222> (76)..(76)
   <223> Xaa at position 76 is Lys or Arg
<220>
   <221> MISC_FEATURE
   <222> (89)..(89)
   <223> Xaa at position 89 is Glu or Asp
<220>
   <221> MISC_FEATURE
   <222> (107)..(107)
   <223> Xaa at position 107 is Leu or Thr
<400> 14
   Xaa Val Gln Leu Val Glu Xaa Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Xaa Leu Val
           35                  40                  45
   Ala Gln Ile Asn Ser Val Gly Xaa Xaa Xaa Tyr Tyr Pro Asp Xaa Val
       50                  55                  60
   Lys Gly Arg Phe Thr Ile Ser Arg Asp Asn Xaa Xaa Asn Thr Leu Tyr
   65                  70                  75                  80
   Leu Gln Met Asn Ser Leu Arg Ala Xaa Asp Thr Ala Val Tyr Tyr Cys
                   85                  90                  95
   Ala Ser Gly Asp Tyr Trp Gly Gln Gly Thr Xaa Val Thr Val Ser Ser
               100                 105                 110
<210> 15
   <211> 112
   <212> PRT
   <213> Artificial Sequence
<220>
   <223> Humanized Antibody
<220>
   <221> MISC_FEATURE
   <222> (1)..(112)
   <223> Deglycosylated Humanized Antibody Heavy Chain Variable Region
<220>
   <221> MISC_FEATURE
   <222> (56)..(56)
   <223> Xaa at position 56 is any amino acid, provided that if Xaa at pos
   ition 57 is neither Asp nor Pro and Xaa at position 59 is Ser or
   Thr, then Xaa at position 56 is not Asn
<220>
   <221> MISC_FEATURE
   <222> (57)..(57)
   <223> Xaa at position 57 is any amino acid, provided that if Xaa at
   position 56 is Asn and Xaa at position 58 is Ser or Thr, then Xaa at
   position 57 is Asp or Pro
<220>
   <221> MISC_FEATURE
   <222> (58)..(58)
   <223> Xaa at position 58 is any amino acid, provided that if Xaa at
   position 56 is Asn and Xaa at position 57 is neither Asp nor Pro,
   then Xaa at position 58 is neither Ser nor Thr
<400> 15
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Glu Leu Val
           35                  40                  45
   Ala Gln Ile Asn Ser Val Gly Xaa Xaa Xaa Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
               100                 105                 110
<210> 16
   <211> 442
   <212> PRT
   <213> Artificial Sequence
<220>
   <223> Humanized Antibody
<220>
   <221> MISC_FEATURE
   <222> (1) .. (442)
   <223> Humanized Antibody Heavy Chain
<220>
   <221> MISC_FEATURE
   <222> (56)..(56)
   <223> Xaa at position 56 is any amino acid, provided that Xaa at positi
   on 57 is neither Asp nor Pro and Xaa at position 59 is Ser or Thr
   , then Xaa at position 56 is not Asn
<220>
   <221> MISC_FEATURE
   <222> (57)..(57)
   <223> Xaa at position 57 is any amino acid, provided that Xaa at position 56 is Asn and Xaa at position 58 is Ser or Thr, then Xaa at
   position 57 is Asp or Pro
<220>
   <221> MISC_FEATURE
   <222> (58)..(58)
   <223> Xaa at position 58 is any amino acid, provided that Xaa at
   position 56 is Asn and Xaa at position 57 is neither Asp nor Pro,
   then Xaa at position 58 is neither Ser nor Thr
<400> 16
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Glu Leu Val
           35                  40                  45
   Ala Gln Ile Asn Ser Val Gly Xaa Xaa Xaa Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Leu Leu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
   325 330 335
   Gln Pro Arg Glu Pro Gln Val Tyr Thr Leu Pro Pro Ser Arg Asp Glu
   340 345 350
   Leu Thr Lys Asn Gln Val Ser Leu Thr Cys Leu Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Tyr Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440

### SEQUENCE LISTING

<110> ELI LILLY AND COMPANY and WASHINGTON UNIVERSITY
<120> ASSAY METHOD FOR ALZHEIMER'S DISEASE
<130> 53-35
<140> EP 02 766 022.4
   <141> 2002-08-16
<150> PCT/US 02/26321
   <151> 2002-08-16
<150> 60/334,987
   <151> 2001-10-23
<150> 60/313,221
   <151> 2001-08-17
<150> 60/313,224
   <151> 2001-08-17
<160> 16
<170> Patent In version 3.1
<210> 1
   <211> 16
   <212> PRT
   <213> Mus sp.
<220>
   <221> MISC_FEATURE
   <222> (1)..(16)
   <223> LIGHT CHAIN CDR1
<400> 1
   Arg Ser Ser Gln Ser Leu Ile Tyr Ser Asp Gly Asn Ala Tyr Leu His
   1               5                   10                  15
<210> 2
   <211> 7
   <212> PRT
   <213> Mus sp.
<220>
   <221> MISC_FEATURE
   <223> LIGHT CHAIN CDR2
<400> 2
   Lys Val Ser Asn Arg Phe Ser
   1               5
<210> 3
   <211> 9
   <212> PRT
   <213> Mus sp.
<220>
   <221> MISC_FEATURE
   <222> (1)..(9)
   <223> LIGHT CHAIN CDR3
<400> 3
   Ser Gln Ser Thr His Val Pro Trp Thr
   1               5
<210> 4
   <211> 5
   <212> PRT
   <213> Mus sp.
<220>
   <221> MISC_FEATURE
   <222> (1)..(5)
   <223> HEAVY CHAIN CDR1
<400> 4
   Arg Tyr Ser Met Ser
   1               5
<210> 5
   <211> 17
   <212> PRT
   <213> Mus sp.
<220>
   <221> MISC_FEATURE
   <222> (1)..(17)
   <223> HEAVY CHAIN CDR2
<400> 5
   Gln Ile Asn Ser Val Gly Asn Ser Thr Tyr Tyr Pro Asp Thr Val Lys
   1               5                   10                  15
   Gly
<210> 6
   <211> 3
   <212> PRT
   <213> Mus sp.
<220>
   <221> MISC_FEATURE
   <222> (1)..(3)
   <223> HEAVY CHAIN CDR3
<400> 6
   Gly Asp Tyr
   1
<210> 7
   <211> 113
   <212> PRT
   <213> Artificial Sequence
<220>
   <223> Humanized antibody
   <220>
   <221> MISC_FEATURE
   <222> (1)..(113)
   <223> HUMANIZED ANTIBODY LIGHT CHAIN VARIABLE REGION
<220>
   <221> MISC_FEATURE
   <222> (88)..(88)
   <223> Xaa at position 88 is Val or Leu
<220>
   <221> MISC_FEATURE
   <222> (105)..(105)
   <223> Xaa at position 105 is Gln or Gly
<220>
   <221> MISC_FEATURE
   <222> (108)..(108)
   <223> Xaa at position 108 is Lys or Arg
<220>
   <221> MISC_FEATURE
   <222> (109)..(109)
   <223> Xaa at position 109 is Val or Leu
<220>
   <221> MISC_FEATURE
   <222> (14) .. (14)
   <223> Xaa at position 14 is Thr or Ser
<220>
   <221> MISC_FEATURE
   <222> (15)..(15)
   <223> Xaa at position 15 is Leu or Pro
<220>
   <221> MISC_FEATURE
   <222> (30)..(30)
   <223> Xaa at position 30 is Ile or Val
<220>
   <221> MISC_FEATURE
   <222> (50)..(50)
   <223> Xaa at position 50 is Arg, Gin, or Lys
<220>
   <221> MISC_FEATURE
   <222> (7)..(7)
   <223> Xaa at position 7 is Ser or Thr
<220>
   <221> MISC_FEATURE
   <222> (2)..(2)
   <223> Xaa at position 2 is Val or Ile
<400> 7
   Asp Xaa Val Met Thr Gln Xaa Pro Leu Ser Leu Pro Val Xaa Xaa Gly
   1               5                   10                  15
   Gln Pro Ala Ser Ile Ser Cys Arg Ser Ser Gln Ser Leu Xaa Tyr Ser
               20                  25                  30
   Asp Gly Asn Ala Tyr Leu His Trp Phe Leu Gln Lys Pro Gly Gln Ser
           35                  40                  45
   Pro Xaa Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Xaa Gly Val Tyr Tyr Cys Ser Gln Ser
                   85                  90                  95
   Thr His Val Pro Trp Thr Phe Gly Xaa Gly Thr Xaa Xaa Glu Ile Lys
               100                 105                 110
   Arg
<210> 8
   <211> 112
   <212> PRT
   <213> Artificial Sequence
<220>
   <223> Humanized antibody
   <220>
   <221> MISC_FEATURE
   <222> (1)..(112)
   <223> HUMANIZED ANTIBODY HEAVY CHAIN VARIABLE REGION
<220>
   <221> MISC_FEATURE
   <222> (76)..(76)
   <223> Xaa at position 76 is Lys or Arg
<220>.
   <221> MISC_FEATURE
   <222> (89)..(89)
   <223> Xaa at position 89 is Glu or Asp
<220>
   <221> MISC_FEATURE
   <222> (107)..(107)
   <223> Xaa at position 107 is Leu or Thr
<220>
   <221> MISC_FEATURE
   <222> (1) .. (1)
   <223> Xaa at position 1 is Glu or GIn
<220>
   <221> MISC_FEATURE
   <222> (7)..(7)
   <223> Xaa at position 7 is Ser or Leu
<220>
   <221> MISC_FEATURE
   <222> (46)..(46)
   <223> Xaa at position 46 is Glu, Val, Asp, or Ser
<220>
   <221> MISC_FEATURE
   <222> (63)..(63)
   <223> Xaa at position 63 is Thr or Ser
<220>
   <221> MISC_FEATURE
   <222> (75)..(75)
   <223> Xaa at position 75 is Ala, Ser, Val, or Thr
<400> 8
   Xaa Val Gln Leu Val Glu Xaa Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Xaa Leu Val
           35                  40                  45
   Ala Gln Ile Asn Ser Val Gly Asn Ser Thr Tyr Tyr Pro Asp Xaa Val
       50                  55                  60
   Lys Gly Arg Phe Thr Ile Ser Arg Asp Asn Xaa Xaa Asn Thr Leu Tyr
   65                  70                  75                  80
   Leu Gln Met Asn Ser Leu Arg Ala Xaa Asp Thr Ala Val Tyr Tyr Cys
                   85                  90                  95
   Ala Ser Gly Asp Tyr Trp Gly Gln Gly Thr Xaa Val Thr Val Ser Ser
               100                 105                 110
<210> 9
   <211> 113
   <212> PRT
   <213> Artificial Sequence
<220>
   <223> Humanized antibody
   <220>
   <221> MISC_FEATURE
   <222> (1)..(113)
   <223> HUMANIZED ANTIBODY LIGHT CHAIN VARIABLE REGION
<400> 9
   Asp Val Val Met Thr Gln Ser Pro Leu Ser Leu Pro Val Thr Leu Gly
   1               5                   10                  15
   Gln Pro Ala Ser Ile Ser Cys Arg Ser Ser Gln Ser Leu Ile Tyr Ser
               20                  25                  30
   Asp Gly Asn Ala Tyr Leu His Trp Phe Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Ser
                   85                  90                  95
   Thr His Val Pro Trp Thr Phe Gly Gln Gly Thr Lys Val Glu Ile Lys
               100                 105                 110
   Arg
<210> 10
   <211> 112
   <212> PRT
   <213> Artificial Sequence
<220>
   <223> Humanized antibody
   <220>
   <221> MISC_FEATURE
   <222> (1)..(112)
   <223> HUMANIZED ANTIBODY HEAVY CHAIN VARIABLE REGION
<400> 10
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Glu Leu Val
           35                  40                  45
   Ala Gln Ile Asn Ser Val Gly Asn Ser Thr Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
               100                 105                 110
<210> 11
   <211> 219
   <212> PRT
   <213> Artificial Sequence
<220>
   <223> Humanized antibody
   <220>
   <221> MISC_FEATURE
   <222> (1)..(219)
   <223> HUMANIZED ANTIBODY LIGHT CHAIN
<400> 11
   Asp Val Val Met Thr Gln Ser Pro Leu Ser Leu Pro Val Thr Leu Gly
   1               5                   10                  15
   Gln Pro Ala Ser Ile Ser Cys Arg Ser Ser Gln Ser Leu Ile Tyr Ser
               20                  25                  30
   Asp Gly Asn Ala Tyr Leu His Trp Phe Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Ser
                   85                  90                  95
   Thr His Val Pro Trp Thr Phe Gly Gln Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 12
   <211> 442
   <212> PRT
   <213> Artificial Sequence
<220>
   <223> Humanized antibody
   <220>
   <221> MISC_FEATURE
   <222> (1)..(442)
   <223> HUMANIZED ANTIBODY HEAVY CHAIN
<400> 12
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Glu Leu Val
           35                  40                  45
   Ala Gln Ile Asn Ser Val Gly Asn Ser Thr Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Leu Leu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
                   325                 330                 335
   Gln Pro Arg Glu Pro Gln Val Tyr Thr Leu Pro Pro Ser Arg Asp Glu
               340                 345                 350
   Leu Thr Lys Asn Gln Val Ser Leu Thr Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Tyr Ser Lys Leu Thr Val Asp Lys Ser Arg Trp Gln Gln Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Pro Gly Lys
           435                 440
<210> 13
   <211> 17
   <212> PRT
   <213> Mouse Variant
<220>
   <221> MISC_FEATURE
   <222> (1)..(17)
   <223> HEAVY CHAIN CDR2
<220>
   <221> MISC_FEATURE
   <222> (7)..(7)
   <223> Xaa at position 7 is any amino acid, provided that is Xaa at position 8 is neither Asp nor Pro and Xaa at position 9 is Ser or Thr, then Xaa at position 7 is not Asn
<220>
   <221> MISC_FEATURE
   <222> (8)..(8)
   <223> Xaa at position 8 is any amino acid, provided that Xaa at position 7 is Asn and Xaa at position 9 is Ser or Thr, then Xaa at position 8 is Asp or Pro
<220>
   <221> MISC_FEATURE
   <222> (9)..(9)
   <223> Xaa at position 9 is any amino acid, provided that Xaa at position 7 is Asn and Xaa at position 8 is neither Asp nor Pro, then Xaa at position 9 is neither Ser nor Thr
<400> 13
   Gln Ile Asn Ser Val Gly Xaa Xaa Xaa Tyr Tyr Pro Asp Thr Val Lys
   1               5                   10                  15
   Gly
<210> 14
   <211> 112
   <212> PRT
   <213> Artificial Sequence
<220>
   <223> Humanized Antibody
<220>
   <221> MISC_FEATURE
   <222> (1).. (112)
   <223> Deglycosylated Humanized Antibody Heavy Chain Variable Region
<220>
   <221> MISC_FEATURE
   <222> (63)..(63)
   <223> Xaa at position 63 is Thr or Ser
<220>
   <221> MISC_FEATURE
   <222> (1)..(1)
   <223> Xaa at position 1 is Glu or Gln
<220>
   <221> MISC_FEATURE
   <222> (7)..(7)
   <223> Xaa at position 7 is Ser or Leu
<220>
   <221> MISC_FEATURE
   <222> (46)..(46)
   <223> Xaa at position 46 is Glu, Val, Asp, or Ser
<220>
   <221> MISC_FEATURE
   <222> (56)..(56)
   <223> Xaa at position 56 is any amino acid, provided that if Xaa at pos ition 57 is neither Asp nor Pro and Xaa at position 59 is Ser or Thr, then Xaa at position 56 is not Asn
<220>
   <221> MISC_FEATURE
   <222> (57)..(57)
   <223> Xaa at position 57 is any amino acid, provided that if Xaa at pos ition 56 is Asn and Xaa at position 58 is Ser or Thr, then Xaa at position 57 is Asp or Pro
<220>
   <221> MISC_FEATURE
   <222> (58)..(58)
   <223> Xaa at position 57 is any amino acid, provided that if Xaa at pos ition 56 is Asn and Xaa at position 57 is neither Asp nor Pro, th en Xaa at position 58 is neither Ser nor Thr
<220>
   <221> MISC_FEATURE
   <222> (75)..(75)
   <223> Xaa at position 75 is Ala, Ser, Val, or Thr
<220>
   <221> MISC_FEATURE
   <222> (76).. (76)
   <223> Xaa at position 76 is Lys or Arg
<220>
   <221> MISC_FEATURE
   <222> (89)..(89)
   <223> Xaa at position 89 is Glu or Asp
<220>
   <221> MISC_FEATURE
   <222> (107)..(107)
   <223> Xaa at position 107 is Leu or Thr
<400> 14
   Xaa Val Gln Leu Val Glu Xaa Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Xaa Leu Val
           35                  40                  45
   Ala Gln Ile Asn Ser Val Gly Xaa Xaa Xaa Tyr Tyr Pro Asp Xaa Val
       50                  55                  60
   Lys Gly Arg Phe Thr Ile Ser Arg Asp Asn Xaa Xaa Asn Thr Leu Tyr
   65                  70                  75                  80
   Leu Gln Met Asn Ser Leu Arg Ala Xaa Asp Thr Ala Val Tyr Tyr Cys
                   85                  90                  95
   Ala Ser Gly Asp Tyr Trp Gly Gln Gly Thr Xaa Val Thr Val Ser Ser
               100                 105                 110
<210> 15
   <211> 112
   <212> PRT
   <213> Artificial Sequence
<220>
   <223> Humanized Antibody
<220>
   <221> MISC_FEATURE
   <222> (1) .. (112)
   <223> Deglycosylated Humanized Antibody Heavy Chain Variable Region
<220>
   <221> FEATURE_FEATURE
   <222> (56) .. (56)
   <223> Xaa at position 56 is any amino acid, provided that if Xaa at pos ition 57 is neither Asp nor Pro and Xaa at position 59 is Ser or Thr, then Xaa at position 56 is not Asn
<220>
   <221> MISC_FEATURE
   <222> (57)..(57)
   <223> Xaa at position 57 is any amino acid, provided that if Xaa at pos ition 56 is Asn and Xaa at position 58 is Ser or Thr, then Xaa at position 57 is Asp or Pro
<220>
   <221> MISC_FEATURE
   <222> (58)..(58)
   <223> Xaa at position 58 is any amino acid, provided that if Xaa at pos ition 56 is Asn and Xaa at position 57 is neither Asp nor Pro, th en Xaa at position 58 is neither Ser nor Thr
<400> 15
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Glu Leu Val
           35                  40                  45
   Ala Gln Ile Asn Ser Val Gly Xaa Xaa Xaa Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
               100                 105                 110
<210> 16
   <211> 442
   <212> PRT
   <213> Artificial Sequence
<220>
   <223> Humanized Antibody
<220>
   <221> MISC_FEATURE
   <222> (1)..(442)
   <223> Humanized Antibody Heavy Chain
<220>
   <221> MISC_FEATURE
   <222> (56)..(56)
   <223> Xaa at position 56 is any amino acid, provided that Xaa at positi on 57 is neither Asp nor Pro and Xaa at position 59 is Ser or Thr then Xaa at position 56 is not Asn
<220>
   <221> MISC_FEATURE
   <222> (57)..(57)
   <223> Xaa at position 57 is any amino acid, provided that Xaa at positi on 56 is Asn and Xaa at position 58 is Ser or Thr, then Xaa at po sition 57 is Asp or Pro
<220>
   <221> MISC_FEATURE
   <222> (58) .. (58)
   <223> Xaa at position 58 is any amino acid, provided that Xaa at positi on 56 is Asn and Xaa at position 57 is neither Asp nor Pro, then Xaa at position 58 is neither Ser nor Thr
<400> 16
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
               20                  25                  30
   Ser Met Ser Trp Val Arg Gln Ala Pro Gly Lys Gly Leu Glu Leu Val
           35                  40                  45
   Ala Gln Ile Asn Ser Val Gly Xaa Xaa Xaa Tyr Tyr Pro Asp Thr Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Leu Val Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Leu Leu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
                   325                 330                 335
   Gln Pro Arg Glu Pro Gln Val Tyr Thr Leu Pro Pro Ser Arg Asp Glu
               340                 345                 350
   Leu Thr Lys Asn Gln Val Ser Leu Thr Cys Leu Val Lys Gly Phe Tyr
           355                 360                 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Tyr Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440

## Description

### Technical Field

The invention relates to an assay which permits diagnosis of preclinical and clinical Alzheimer's disease. The test relies on assessing the levels of amyloid beta (A13) peptide in plasma following administration of certain anti-Aβ antibodies to a subject.

### Background Art

A number of symptomologies which result in cognitive deficits, stroke, brain hemorrhage, and general mental debilitation appear to be associated with neuritic and cerebrovascular plaques in the brain containing the amyloid beta peptide (Aβ).
Among these conditions are both preclinical and clinical Alzheimer's disease, Down's syndrome, and preclinical and clinical cerebral amyloid angiopathy (CAA). The amyloid plaques are formed from amyloid beta peptides. These peptides circulate in the blood and in the cerebrospinal fluid (CSF). The Aβ peptide in circulating form is composed of 39-43 amino acids (mostly 40 or 42 amino acids) resulting from the cleavage of a common precursor protein, amyloid precursor protein, often designated APP.

Evidence suggests that Aβ can be transported back and forth between brain and the blood (Ghersi-Egea, J-F., et al., J. Neurochem. (1996) 67:880-883; Zlokovic, B.V., et al., Biochem. Biophys. Res. Comm. (1993) 67:1034-1040; Shibata, M., et al., J. Clin. Invest. (2000)106:1489-1499. Further Aβ in plaques is in an equilibrium with soluble Aβ in the brain and blood (Kawarabayashi, T., et al., J Neurosci. (2001) 21:372-381), DeMattos et al., Proc. Nat'l.Acad Sci USA (2001) 98:8850-8855.

As described in WO01/4987, total circulating levels of Aβ peptide in CSF are similar in normal individuals and individuals predisposed to exhibit the symptoms of Alzheimer's. However, Aβ₄₂ levels are lower on average in individuals with Alzheimer's disease (Nitsch,R.M., et al., Ann. Neurol. (1995) 37:512-518). It is known that Aβ₄₂ is more prone to aggregate than is Aβ₄₂, and when this happens, adverse consequences such as Aβ deposition in amyloid plaques, conversion of Aβ to toxic forms, nerve cell damage, and behavioral impairment such as dementia ensue (Golde, T.E., et al., Biochem. Biophys. Acta. (2000) 1502:172-187).

WO01/62801 entitled "Humanized Antibodies That Sequester Aβ Peptide" filed 26 February 2001 describes antibodies which do not appreciably cross the blood-brain barrier and which sequester Aβ peptides circulating in biological fluids. These antibodies are described as useful for preventive and therapeutic treatment of conditions associated with the formation of Aβ-containing diffuse, neuritic, and cerebrovascular plaques in the brain. The application describes administering the antibodies and then measuring circulating levels of Aβ peptide in blood in order to assess the progress of therapy. There is no clear suggestion, however, that the levels of Aβ peptide following administration of the antibodies are diagnostic of the condition itself. The present invention resides in the surprising result that enhanced levels of both Aβ₄₀ and Aβ₄₂ as well as the Aβ₄₀/Aβ₄₂ ratio correlate with the levels of Aβ peptide deposition in the brain when the antibodies have been administered to an individual. Thus, measurement of these components in the blood after administration of the antibody provides a simple straightforward diagnostic test for both clinical and preclinical Alzheimer's disease and related neurological disorders.

There are additional relevant publications concerning the behaviour of Aβ peptide antibodies. For example, PCT publication W099/27944 published 10 June 1999 describes methods to induce an immune response in order to reduce amyloid deposits. Publication No. W099/60024 published 25 November 1999, describes methods for amyloid removal using anti-amyloid antibodies. Additional PCT publications, including WO00/72880, WO00/72876 and WO00/77178 all describe various activities of anti-Aβ peptide antibodies. Antibodies directed to the N-terminus of this peptide are said to reduce plaques in a transgenic murine model; immunization with the amyloid itself is described as are antibodies designed to catalyze hydrolysis of the peptide.

It has been shown that one pathway for Aβ metabolism is via transport from CNS to the plasma (Zlokovic, B.V., et al., Proc. Natl. Acad. Sci (USA) (1996) 93:4229-4234;Ghersi-Egea, J-F., et al., J. Neurochem.(1996)67:880-883). Additionally, it has been shown that Aβ in plasma can cross the blood-brain-barrier and enter the brain (Zlokovic, B.V., et al., Biochem. Biophys. Res. Comm. (1993) 67:1034-1040). It has also been shown that administration of certain polyclonal and monoclonal Aβ antibodies decreases Aβ deposition in amyloid plaques in the APP^{V717F} transgenic mouse model of Alzheimer's disease (Bard, F., et al., Nature Med. (2000) 6:916-919). This was said to be due to certain anti-Aβ antibodies crossing the blood-brain-barrier and stimulating phagocytosis of amyloid plaques by microglial cells. In Bard's experiments, assays of brain slices *ex vivo* showed that the presence of added Aβ antibody, along with exogenously added microglia, induced phagocytosis of Aβ, resulting in removal of Aβ deposits.

The levels of both soluble Aβ₄₀ and Aβ₄₂ in CSF and blood can readily be detected using standardized assays using antibodies directed against epitopes along the Aβ chain. Such assays have been reported, for example, in U.S. patents 5,766,846; 5,837,672; and 5,593,846. These patents describe the production of murine monoclonal antibodies to the central domain of the Aβ peptide, and these were reported to have epitopes around and including positions 16 and 17. Antibodies directed against the N-terminal region were described as well. Several monoclonal antibodies were asserted to immunoreact with positions 13-28 of the Aβ peptide; these did not bind to a peptide representing positions 17-28, thus, according to the cited patents, establishing that it is this region, including positions 16-17 (the 0-secretase site) that was the target of these antibodies. Among antibodies known to bind between amino acids 13 and 28 of Aβ are mouse antibodies 266 (m266), 4G8, and 1C2.

### Disclosure of the Invention

It has now been found that antibodies which are useful for performing assays for Aβ peptide, and which are useful in treatment of conditions associated with amyloid plaques in the brain can elicit a response which results in a marked increase in the level of Aβ peptide in the blood and this level can be used as a diagnostic marker for clinical and preclinical Alzheimer's disease. These antibodies, which may or may not be humanized, sequester Aβ peptide from its bound, circulating form in blood and alter clearance of soluble and bound forms of Aβ in central nervous system and plasma. These antibodies, and fragments thereof, specifically bind to an epitope between amino acids 13 and 28 of the Aβ molecule. The CDR of these antibodies can be derived from mouse monoclonal antibody 266 (SEQ ID NO: 1 through SEQ ID NO:6). Useful antibodies include antibodies and fragments thereof, wherein the variable regions have sequences comprising the CDR from mouse antibody 266 and specific human framework sequences (SEQ ID NO:7 through SEQ ID NO:10), wherein the antibodies retain approximately the binding properties of the mouse antibody and have *in vitro* and *in vivo* properties functionally equivalent to the mouse antibody 266. Especially useful are humanized antibodies and fragments thereof, wherein the light chain is SEQ ID NO:11 and the heavy chain is SEQ ID NO:12.

Thus in one aspect, the invention is directed to a method of diagnosing preclinical or clinical Alzheimer's disease comprising:
(a) measuring one or more of
   (i) the level of Aβ₄₀;
   (ii) the level of Aβ₄₂; or
   (ii) the ratio Aβ₄₀/Aβ₄₂;
   in a blood sample of a subject obtained at a time interval after administering to said subject an amount of an antibody which specifically binds an epitope contained within positions 13-28 of Aβ or an antibody that sequesters Aβ peptide from its bound, circulating form in the blood and alters clearance of soluble and bound forms of Aβ in the central nervous system in plasma, wherein said amount is effective to alter the levels of circulating Aβ peptides in the blood of said subject when said subject is in a preclinical or clinical stage of Alzheimer's disease; and
(b) comparing the level of Aβ₄₀, Aβ₄₂, or the ratio of Aβ₄₀/Aβ₄₂ in said subject with a control value of said levels, wherein elevated levels of Aβ₄₀, Aβ₄₂, or the ratio of Aβ₄₀/Aβ₄₂ in said subject as compared to control levels or ratio identifies said subject as in a preclinical or clinical stage of Alzheimer's disease.

### Brief Description of the Drawings

Figures 1 A, B and C are graphs showing the levels of Aβ₄₀ (Figure 1A), Aβ₄₂ (Figure 1B), and Aβ₄₀/Aβ₄₂ ratio (Figure 1C) in plasma of transgenic mice prior to administration of the antibody m266, and the lack of correlation with brain Aβ deposits.
Figures 2 A and B are graphs showing plasma Aβ₄₀ (Figure 2A) and plasma Aβ₄₀/Aβ₄₂ ratio (Figure 2B) in transgenic mice one hour after injection of antibody m266, and the significant correlation with brain Aβ deposits.
Figures 3 A, B and C are graphs showing the significant correlations of the two Aβ peptides (Figures 3A and 3B) and their ratio (Figure 3C) with Aβ peptide deposition in the brain 24 hours after injection with monoclonal antibody m266.
Figures 4 A, B and C are graphs showing the significant correlations of entry rates into the circulation of the two Aβ peptides (Figures 4A and 4B) and their ratio (Figure 4C) and Aβ peptide deposition in transgenic mice.
Figures 5 A and B are graphs showing an alternative graphical representation of Aβ₄₀ levels in the plasma 24 hours (Figure 5A) and 1 hour (Figure 5B) after m266 injection correlated with the percentage hippocampus covered by Aβ deposits.
Figure 6 is a table showing Pearson correlation coefficients (Pearson r) and significance (P value) determined between plasma Aβ values (pre and post injection of m266) and hippocampal Aβ or amyloid load.

### Modes of Carrying Out the Invention

The Aβ peptides that circulate in human biological fluids represent a carboxy terminal region of a precursor protein encoded on chromosome 21. It has been reported from the results of *in vitro* experiments that the Aβ peptide has poor solubility in physiological solutions, since it contains a stretch of hydrophobic amino acids which are a part of the region that anchors its longer precursor to the lipid membranes of cells. It is thus not surprising that circulating Aβ peptide is normally complexed with other moieties that prevent it from aggregating. This has resulted in difficulties in detecting circulating Aβ peptide in biological fluids.

The above-mentioned patent documents (U.S. patents 5,766,846; 5,837,672 and 5,593,846) describe the preparation of antibodies, including a monoclonal antibody, designated clone 266 (m266), which was raised against, and has been shown to bind specifically to, a peptide comprising amino acids 13-28 of the Aβ peptide. Applicants have found that after administering m266 to APP^{V717F} mice, a mouse model of Alzheimer's disease, they can measure levels of Aβ peptides in the circulation that are diagnostic of the levels of amyloid plaques in the brain. Thus, these antibodies are useful not only in conducting assays for circulating Aβ peptides *per se,* but also for eliciting circulating blood levels which are diagnostic of the amount of amyloid plaque in the brain, and thus useful in identifying individuals in clinical and preclinical stages of Alzheimer's disease. One such antibody, m266, bonds to the mid-region of Aβ peptide.

By "monoclonal antibody that bonds to the mid-region of Aβ peptide" is meant a monoclonal antibody (Mab or Mabs) that binds an amino acid sequence representing an epitope contained between positions 13-28 of Aβ. The entire region need not be targeted. As long as the antibody binds at least an epitope within this region (especially, *e.g.,* including the α-secretase site 16-17 or the site-at which antibody 266 binds), such antibodies are effective in the method of the invention.

By "antibody" is meant a monoclonal antibody per *se,* or an immunologically effective fragment thereof, such, as an F_{ab}, F_{ab'}, or F(_{ab'})₂ fragment thereof. In some contexts, herein, fragments will be mentioned specifically for emphasis; nevertheless, it will be understood that regardless of whether fragments are specified, the term "antibody" includes such fragments as well as single-chain forms. As long as the protein retains the ability specifically to bind its intended target, and in this case, to sequester Aβ peptide from its carrier proteins in blood, it is included within the term "antibody." Also included within the definition "antibody" for example, are single chain forms, generally designated Fᵥ, regions, of antibodies with this specificity. Preferably, but not necessarily, the antibodies useful in the invention are produced recombinantly, as manipulation of the typically murine or other non-human antibodies with the appropriate specificity is required in order to convert them to humanized form. Antibodies may or may not be glycosylated, though glycosylated antibodies are preferred. Antibodies are properly cross-linked via disulfide bonds, as is well-known.

The basic antibody structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function.

Light chains are classified as gamma, mu, alpha, and lambda. Heavy chains are classified as gamma, mu, alpha, delta, or epsilon, and define the antibody's isotype as IgG, IgM, IgA, IgD and IgE, respectively. Within light and heavy chains, the variable and constant regions are joined by a "J" region of about 12 or more amino acids, with the heavy chain also including a "D" region of about 10 more amino acids.

The variable regions of each light/heavy chain pair form the antibody binding site. Thus, an intact antibody has two binding sites. The chains all exhibit the same general structure of relatively conserved framework regions (FR) joined by three hypervariable regions, also called complementarily determining regions or CDRs. The CDRs from the two chains of each pair are aligned by the framework regions, enabling binding to a specific epitope. From N-terminal to C-terminal, both light and heavy chains comprise the domains FR1, CDR1, FR2,CDR2, FR3, CDR3 and FR4. The assignment of amino acids to each domain is in accordance with well known conventions [Kabat "Sequences of Proteins of Immunological Interest" National Institutes of Health, Bethesda, Md., 1987 and 1991; Chothia, et al., J. Mol. Bio. (1987)196:901-917; Chothia, et al., Nature (1989) 342:878-883].

As is well understood in the art, monoclonal antibodies can readily be generated with appropriate specificity by standard techniques of immunization of mammals, forming hybridomas from the antibody-producing cells of said mammals or otherwise immortalizing them, and culturing the hybridomas or immortalized cells to assess them for the appropriate specificity. In the present case such antibodies could be generated by immunizing a human, rabbit, rat or mouse, for example, with a peptide representing an epitope encompassing the 13-28 region of the Aβ peptide or an appropriate subregion thereof. Materials for recombinant manipulation can be obtained by retrieving the nucleotide sequences encoding the desired antibody from the hybridoma or other cell that produces it. These nucleotide sequences can then be manipulated to provide them in humanized form, if desired.

It may be desirable to utilize humanized forms of these antibodies in order to elicit the desired circulating levels of the peptides in human subjects. Since the administration is short-term and only for diagnostic purposes, this may not be necessary, but clearly it is preferable to avoid any possibility of an immune response, so the use of humanized forms for this purpose is preferred. Of course, for the performance of the assay of Aβ levels *ex vivo* (e.g. by ELISA), the murine forms themselves can be used.

By "humanized antibody" is meant an antibody that is composed partially or fully of amino acid sequences derived from a human antibody germline by altering the sequence of an antibody having non-human complementarity determining regions (CDR). The simplest such alteration may consist simply of substituting the constant region of a human antibody for the murine constant region, thus resulting in a human/murine chimera which may have sufficiently low immunogenicity to be acceptable for pharmaceutical use. Preferably, however, the variable region of the antibody and even the CDR is also humanized by techniques that are by now well known in the art. The framework regions of the variable regions are substituted by the corresponding human framework regions leaving the non-human CDR substantially intact, or even replacing the CDR with sequences derived from a human genome. Fully human antibodies are produced in genetically modified mice whose immune systems have been altered to correspond to human immune systems. As mentioned above, it is sufficient for use in the methods of the invention, to employ an immunologically specific fragment of the antibody, including fragments representing single chain forms.

A humanized antibody thus refers to an antibody comprising a human framework, at least one CDR from a non-human antibody, and in which any constant region present is substantially identical to a human inimunoglobulin constant region, *i.e*., at least about 85-90%, preferably at least 95% identical. Hence, all parts of a humanized antibody, except possibly the CDRs, are substantially identical to corresponding parts of one or more native human immunoglobulin sequences. For example, a humanized immunoglobulin would typically not encompass a chimeric mouse variable region/human constant region antibody.

The design of humanized immunoglobulins may be carried out as follows. When an amino acid falls under the following category, the framework amino acid of a human immunoglobulin to be used (acceptor immunoglobulin) is replaced by a framework amino acid from a CDR-providing non-human immunoglobulin (donor immunoglobulin):(a) the amino acid in the human framework region of the acceptor immunoglobulin is unusual for human immunoglobulin at that position, whereas the corresponding amino acid in the donor immunoglobulin is typical for human immunoglobulin at that position;(b) the position of the amino acid is immediately adjacent to one of the CDRs; or(c) any side chain atom of a framework amino acid is within about 5-6 angstroms (center-to-center) of any atom of a CDR amino acid in a three dimensional immunoglobulin model [*Queen, et al., op. cit.,* and Co, et al., Proc. Natl. Acad. Sci. USA (1991) 88:2869]. When each of the amino acid in the human framework region of the acceptor immunoglobulin and a corresponding amino acid in the donor immunoglobulin is unusual for human immunoglobulin at that position, such an amino acid is replaced by an amino acid typical for human immunoglobulin at that position.

A preferred humanized antibody is a humanized form of mouse antibody 266. The CDRs of humanized 266 have the following amino acid sequences:
light chain CDR1 :
light chain CDR2:
light chain CDR3:
heavy chain CDR1 :
heavy chain CDR2:
and, heavy chain CDR3:

A preferred light chain variable region of a humanized antibody of the present invention has the following amino acid sequence, in which the framework originated from human germline Vk segments DPK18 and J segment Jkl, with several amino acid substitutions to the consensus amino acids in the same human V subgroup to reduce potential immunogenicity: wherein:
Xaa at position 2 is Val or IIe;
Xaa at position 7 is Ser or Thr;
Xaa at position 14 is Thr or Ser;
Xaa at position 15 is Leu or Pro;
Xaa at position 30 is Ile or Val;
Xaa at position 50 is Arg, Gln, or Lys;
Xaa at position 88 is Val or Leu;
Xaa at position 105 is Gln or Gly;
Xaa at position 108 is Lys or Arg; and
Xaa at position 109 is Val or Leu.

A preferred heavy chain variable region of a humanized antibody has the following amino acid sequence, in which the framework originated from human germline VH segments DP53 and J segment JH4, with several amino acid substitutions to the consensus amino acids in the same human subgroup to reduce potential immunogenicity: wherein:
Xaa at position 1 is Glu or Gln;
Xaa at position 7 is Ser or Leu;
Xaa at position 46 is Glu, Val, Asp, or Ser;
Xaa at position 63 is Thr or Ser;
Xaa at position 75 is Ala, Ser, Val, or Thr;
Xaa at position 76 is Lys or Arg;
Xaa at position 89 is Glu or Asp; and
Xaa at position 107 is Leu or Thr.

A particularly preferred light chain variable region of a humanized antibody has the following amino acid sequence, in which the framework originated from human germline Vk segments DPK18 and J segment Jkl, with several amino acid substitutions to the consensus amino acids in the same human V subgroup to reduce potential immunogenicity:

A particularly preferred heavy chain variable region of a humanized antibody has the following amino acid sequence, in which the framework originated from human germline VH segments DP53 and J segment JH4:

A preferred light chain for a humanized antibody has the amino acid sequence:

A preferred heavy chain for a humanized antibody has the amino acid sequence:

Other sequences are possible for the light and heavy chains for the humanized antibodies and for humanized 266. The immunoglobulins can have two pairs of light chain/heavy chain complexes, at least one chain comprising one or more mouse complementarity determining regions functionally joined to human framework region segments.

Starting at position 56 of the heavy chain variable region, both m266 and humanized 266 contain the sequence Asn-Ser-Thr. This sequence is an example of the Asn-X-Ser/Thr signal for N-linked glycosylation, wherein the Asn is the site of attachment of N-linked glycosyl chains. Both m266 and humanized 266 are extensively glycosylated at this site. Quite unpredictably and advantageously, the affinity of humanized 266 that is deglycosylated in the heavy chain CDR2 for Aβ peptide is markedly higher than that of humanized 266. The heavy chain CDR2 of deglycosylated humanized 266 has the following amino acid sequences:
heavy chain CDR2:
wherein:
Xaa at position 7 is any amino acid, provided that if Xaa at position 8 is neither Asp nor Pro and Xaa at position 9 is Ser or Thr, then Xaa at position 7 is not Asn;
Xaa at position 8 is any amino acid, provided that if Xaa at position 7 is Asn and Xaa at position 9 is Ser or Thr, then Xaa at position 8 is Asp or Pro; and
Xaa at position 9 is any amino acid, provided that if Xaa at position 7 is Asn and Xaa at position 8 is neither Asp nor Pro, then Xaa at position 9 is neither Ser nor Thr;
By "any amino acid" is meant any naturally-occurring amino acid. Preferred naturally-occurring amino acids are Ala, Cys, Asp, Glu, Phe, Gly, His, Ile, Lys, Leu, Met, Asn, Pro, Gln, Arg, Ser, Thr, Val, Trp, and Tyr.

A preferred deglycosylated humanized antibody is a humanized form of m266, wherein the deglycosylated heavy chain CDR2 is SEQ ID NO:13, wherein:
Xaa at position 7 of SEQ ID NO:13 is selected from the group consisting of Ala, Cys, Asp, Glu, Phe, Gly, His, Ile, Lys, Leu, Met, Asn, Pro, Gln, Arg, Ser, Thr, Val, Trp, and Tyr, provided that if Xaa at position 8 is neither Asp nor Pro and Xaa at position 9 is Ser or Thr, then Xaa at position 7 is not Asn;
Xaa at position 8 of SEQ ID NO:13 is selected from the group consisting of Ala, Cys, Asp, Glu, Phe, Gly, His, Ile, Lys, Leu, Met, Asn, Pro, Gln, Arg, Ser, Thr, Val, Trp, and Tyr, provided that if Xaa at position 7 is Asn and Xaa at position 9 is Ser or Thr, then Xaa at position 8 is Asp or Pro; and
Xaa at position 9 of SEQ ID NO:13 is selected from the group consisting of Ala, Cys, Asp, Glu, Phe, Gly, His, Ile, Lys, Leu, Met, Asn, Pro, Gln, Arg, Ser, Thr, Val, Trp, and Tyr, provided that if Xaa at position 7 is Asn and Xaa at position 8 is neither Asp nor Pro, then Xaa at position 9 is neither Ser nor Thr.

A preferred heavy chain variable region of a deglycosylated humanized antibody has the following amino acid sequence, in which the framework originated from human germline VH segment DP53 and J segment JH4, with several amino acid substitutions to the consensus amino acids in the same human subgroup to reduce potential immunogenicity and wherein the N-glycosylation site in heavy chain CDR2 is modified so that it cannot be N-glycosylated: wherein:
Xaa at position 1 is Glu or Gln;
Xaa at position 7 is Ser or Leu;
Xaa at position 46 is Glu, Val, Asp, or Ser;
Xaa at position 56 is any amino acid, provided that if Xaa at position 57 is neither Asp nor Pro and Xaa at position 59 is Ser or Thr, then Xaa at position 56 is not Asn;
Xaa at position 57 is any amino acid, provided that if Xaa at position 56 is Asn and Xaa at position 58 is Ser or Thr, then Xaa at position 57 is Asp or Pro; and
Xaa at position 58 is any amino acid, provided that if Xaa at position 56 is Asn and Xaa at position 57 is neither Asp nor Pro, then Xaa at position 58 is neither Ser nor Thr
Xaa at position 63 is Thr or Ser;
Xaa at position 75 is Ala, Ser, Val, or Thr;
Xaa at position 76 is Lys or Arg;
Xaa at position 89 is Glu or Asp; and
Xaa at position 107 is Leu or Thr.

A particularly preferred heavy chain variable region of a deglycosylated humanized antibody has the following amino acid sequence, in which the framework originated from human germline VH segment DP53 and J segment JH4 and wherein the N-glycosylation site in heavy chain CDR2 is modified so that it cannot be N-glycosylated: wherein:
Xaa at position 56 is any amino acid, provided that if Xaa at position 57 is neither Asp nor Pro and Xaa at position 59 is Ser or Thr, then Xaa at position 56 is not Asn;
Xaa at position 57 is any amino acid, provided that if Xaa at position 56 is Asn and Xaa at position 58 is Ser or Thr, then Xaa at position 57 is Asp or Pro; and
Xaa at position 58 is any amino acid, provided that if Xaa at position 56 is Asn and Xaa at position 57 is neither Asp nor Pro, then Xaa at position 58 is neither Ser nor Thr.

A preferred heavy chain for a deglycosylated humanized antibody wherein the N-glycosylation site in heavy chain CDR2 is modified so that it cannot be N-glycosylated, has the amino acid sequence: wherein:
Xaa at position 56 is any amino acid, provided that ifXaa at position 57 is neither Asp nor Pro and Xaa at position 59 is Ser or Thr, then Xaa at position 56 is not Asn;
Xaa at position 57 is any amino acid, provided that if Xaa at position 56 is Asn and Xaa at position 58 is Ser or Thr, then Xaa at position 57 is Asp or Pro; and
Xaa at position 58 is any amino acid, provided that if Xaa at position 56 is Asn and Xaa at position 57 is neither Asp nor Pro, then Xaa at position 58 is neither Ser nor Thr.

Preferred deglycosylated 266 antibodies having the heavy variable region according to SEQ ID NO: 14, SEQ ID NO:15, and SEQ ID NO: 16 are those wherein:
Xaa at position 56 is selected from the group consisting of Ala, Gly, His, Asn, Gln, Ser, and Thr, provided that if Xaa at position 58 is Ser or Thr, then Xaa at position 56 is not Asn;
Xaa at position 57 is selected from the group consisting of Ala, Gly, His, Asn, Gin, Ser, and Thr; and
Xaa at position 58 is selected from the group consisting of Ala, Gly, His, Asn, Gln, Ser, and Thr, provided that if Xaa at position 56 is Asn, then Xaa at position 58 is neither Ser nor Thr.

Preferred sequences for CDR2 (positions 56, 57, and 58) of the heavy chain SEQ ID NO: 14, SEQ ID NO:15, and SEQ ID NO:16 include those in which only a single amino acid is changed, those in which only two amino acids are changed, or all three are changed. It is preferred to replace Asn at position 56. It is preferred to replace Thr at position 58 with an amino acid other than Ser. It is preferred to not destroy the N-glycosylation site in the CDR2 of the 266 heavy chain by replacing Ser at position 57 with Pro or Asp. Conservative substitutions at one, two, or all three positions are preferred. The most preferred species are those in which Asn at position 56 is replaced with Ser or Thr. Particularly preferred antibodies are those in which Ser or Thr is at position 56, Ser is at position 57, and Thr is at position 58 of SEQ ID NO:14, SEQ ID NO:15, or SEQ ID NO:16.

Especially preferred deglycosylated species are antibodies comprising a light chain of SEQ ID NO:11 and a heavy chain of SEQ ID NO:16, wherein in SEQ ID N0:16, Xaa at position 56 is Ser, Xaa at position 57 is Ser, and Xaa at position 58 is Thr ("N56S"), or wherein in SEQ ID NO:16, Xaa at position 56 is Thr, Xaa at position 57 is Ser, and Xaa at position 58 is Thr ("N56T").

Production of the antibodies useful in the invention typically involves recombinant techniques, as is described in WO01/62801 cited above.

The antibodies (including immunologically reactive fragments) are administered to a subject to be evaluated for conditions associated with Aβ deposits such as clinical or preclinical Alzheimer's disease, or clinical or preclinical amyloid angiopathy, using standard administration techniques, preferably peripherally (i.e. not by administration into the central nervous system) by intravenous, intraperitoneal, subcutaneous, pulmonary, transdermal, intramuscular, intranasal, buccal, sublingual, or suppository administration.

The compositions for administration are designed to be appropriate for the selected mode of administration, and pharmaceutically acceptable excipients such as dispersing agents, buffers, surfactants, preservatives, solubilizing agents, isotonicity agents, stabilizing agents and the like are used as appropriate. Remington's Pharmaceutical Sciences, Mack Publishing Co., Easton PA, latest edition, provides a compendium of formulation techniques as are generally known to practitioners. It may be particularly useful to alter the solubility characteristics of the antibodies making them more lipophilic, for example, by encapsulating them in liposomes or by blocking polar groups.

Peripheral systemic delivery by intravenous or intraperitoneal or subcutaneous injection is preferred. Suitable vehicles for such injections are straightforward. In addition, however, administration may also be effected through the mucosal membranes by means of nasal aerosols or suppositories. Suitable formulations for such modes of administration are well known and typically include surfactants that facilitate cross-membrane transfer. Such surfactants are often derived from steroids or are cationic lipids, such as N-[1-(2,3-dioleoyl)propyl]-N,N,N-trimethyl ammonium chloride (DOTMA) or various compounds such as cholesterol hemisuccinate, phosphatidyl glycerols and the like.

The concentration of the humanized antibody in formulations from as low as about 0.1% to as much as 15 or 20% by weight and will be selected primarily based on fluid volumes, viscosities, and so forth, in accordance with the particular mode of administration selected. Thus, a typical composition for injection could be made up to contain 1 mL sterile buffered water of phosphate buffered saline and 1-1000 mg, preferably 10-100 mg, of the humanized antibody. The formulation could be sterile filtered after making the formulation, or otherwise made microbiologically acceptable. A typical composition for intravenous infusion could have volumes between 1-250 mL of fluid, such as sterile Ringer's solution, and 1-100 mg per mL, or more in antibody concentration. Therapeutic agents can be frozen or lyophilized for storage and reconstituted in a suitable sterile carrier prior to use. Lyophilization and reconstitution can lead to varying degrees of antibody activity loss (e.g. with conventional immune globulins, IgM antibodies tend to have greater activity loss than IgG antibodies). Dosages may have to be adjusted to compensate. The pH of the formulation will be selected to balance antibody stability (chemical and physical) and comfort to the patient when administered. Generally, pH between 4 and 8 is tolerated.

Although the foregoing methods appear the most convenient and most appropriate for administration of proteins such as humanized antibodies, by suitable adaptation, other techniques for administration, such as transdermal administration and oral administration may be employed provided proper formulation is designed.

In addition, it may be desirable to employ controlled release formulations using biodegradable films and matrices, or osmotic mini-pumps, or delivery systems based on dextran beads, alginate, or collagen.

In summary, formulations are available for administering the antibodies and are well-known in the art and may be chosen from a variety of options.

Typical dosage levels can be optimized using standard clinical techniques and will be dependent on the mode of administration.

After administration of the antibody to the subject, blood samples are withdrawn at periodic intervals over the succeeding minutes, hours, or days. Suitable time periods may be as short as a few minutes, 10 minutes, 30 minutes, or 1 hour, several hours, or days may be allowed to elapse before withdrawal of the blood sample. Measurement after less than 3 hours is preferred. If desired, the plasma fraction can be obtained for ease of analysis. Standard analytic techniques for analysis of the Aβ₄₀, Aβ₄₂ and the ratio thereof are used. These techniques are described, for example, in U.S. patent 5,766,846. Any suitable technique for analysis, however, can be employed, such as chromatographic separation, Western blotting, ELISA assays, homogenous assays and the like.

The concentration of the Aβ₄₀, Aβ₄₂, or their ratio is then compared to these values in a control. Typical controls include individuals known to be free of conditions associated with the amyloid plaques, such as teenagers or very young adults and in addition, age-matched cognitively normal controls are obtained by averaging values from the general population. While some elderly age-matched cognitively normal controls have pre-clinical AD, most do not. Thus, the average values from such a population will be useful and critical to obtain. Design of standard controls is a process that is well known to the ordinary practitioner. Individuals who have elevated levels of the stated peptides or of the ratio of Aβ₄₀ to Aβ₄₂ as compared to the control values are then identified as having a high likelihood of clinical or preclinical conditions associated with the formation of amyloid plaques.

The following examples are intended to illustrate but not to limit the invention.

The examples hereinbelow employ, among others, a murine monoclonal antibody designated "266" which was originally prepared by immunization with a peptide comprised of residues 13-28 of human Aβ peptide. The antibody was confirmed to immunoreact with this peptide, but had previously been reported to not react with the peptide containing only residues 17-28 of human Aβ peptide, or at any other epitopes within the Aβ peptide. The preparation of this antibody is described in U.S. patent 5,766,846. As the examples here describe experiments conducted in murine systems, the use of murine monoclonal antibodies is satisfactory. However, humanized forms of the antibodies with the immunospecificity corresponding to that of antibody 266 are preferred.

### Example 1

### Correlation of Circulating Peptide Levels with Plaques

A murine model for Alzheimer's disease, APP V717F transgenic mice, was used in this assay. These mice are described by Games, D., et al., Nature (1995) 373:523-527; Bales, K.R., et al., Nature Genet. (1997) 17:263-264; and by Holtzman, D.M., et al., Proc. Natl. Acad. Sci. U.S.A. (2000) 97:2892-2897. In this model, a mutant form of the human APP gene is expressed and results in an early onset form of familial Alzheimer's disease. Although the brains of these mice appear normal initially, Aβ deposition in the form of diffuse and neuritic plaques occurs at 6-15 months, although mice homozygous for the transgene show variability in that at 9-14 months of age, some mice develop Aβ deposits while others do not.

53 homozygous mice at 12 months were used in this study.

Plasma levels of Aβ₄₀, Aβ₄₂, and Aβ₄₀/Aβ₄₂ ratios were measured by ELISA in the plasma of these mice prior to administration of 500 µg of m266 and at various time intervals up to 24 hours after administering this antibody. After 24 hours, the mice were sacrificed, and the amount of Aβ deposition in the brain was assessed in the hippocampus and cortex as described by DeMattos, et al. Proc. Nat'l. Acad. Sci USA (2001) 98:8850-8855, and evaluated as a percentage of brain covered by Aβ deposits.

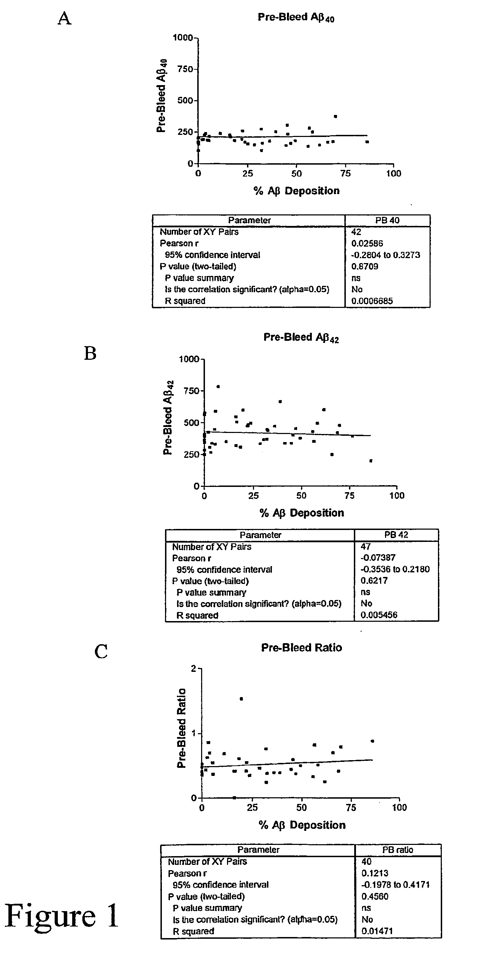

As shown in Figures 1 A, B and C, if the percentage Aβ coverage due to deposition in the hippocampus is plotted on the x-axis against the levels of the peptides and their ratio in plasma on the y-axis prior to administration of the antibody, no correlation is found. Regardless of whether the percent Aβ deposition was essentially zero (0) or over 75%, the average level of Aβ₄₀ was approximately 250 (pg/ml) and of Aβ₄₂ approximately 400 (pg/ml). The ratio of Aβ₄₀ to Aβ₄₂ was thus approximately 0.5-0.6.

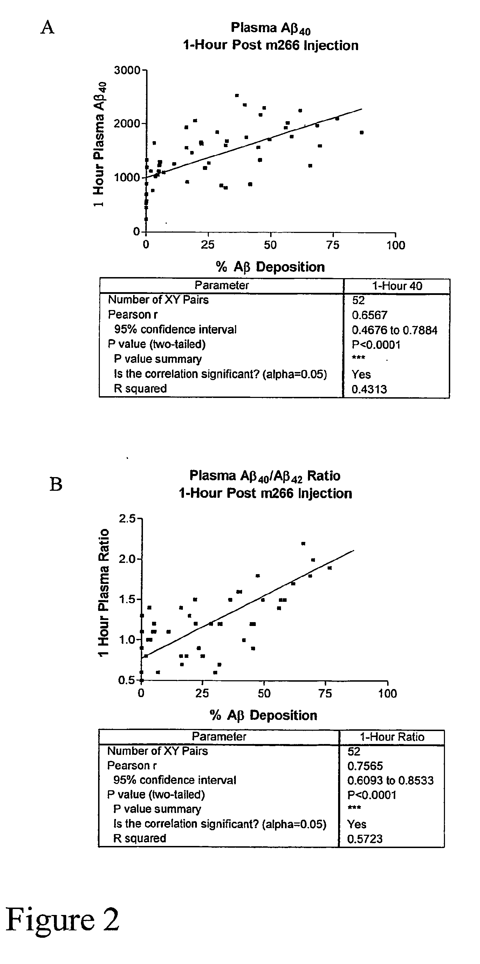

As shown in Figures 2 A and B, however, the plasma level of Aβ40 strongly correlated with the percentage of Aβ deposition in hippocampus one hour after m266 injection, as did the ratio ofAβ₄₀ to Aβ₄₂.

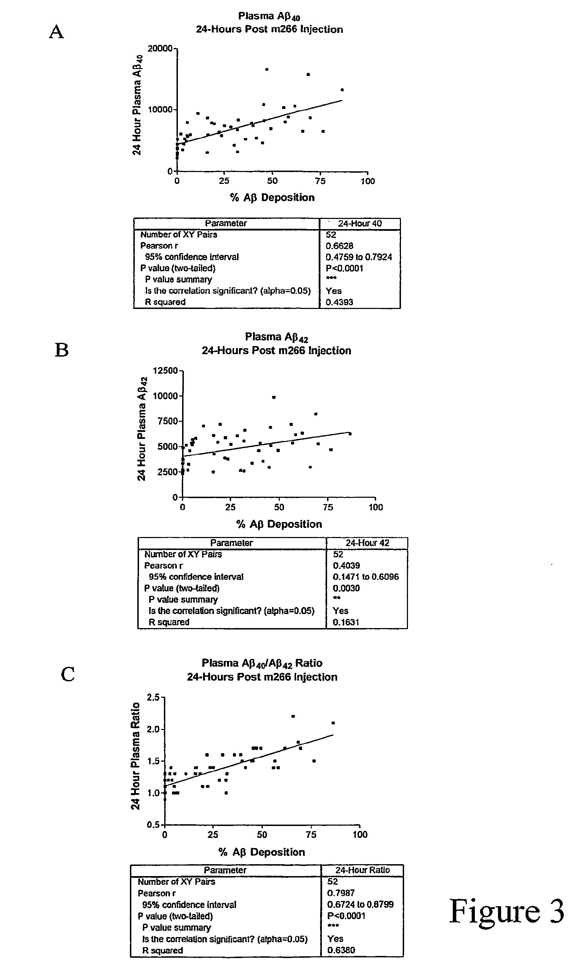

Figures 3 A, B and C show similar results obtained 24 hours post injection. The levels obtained of Aβ₄₀ and the Aβ₄₀/Aβ₄₂ ratio strongly correlated with the % Aβ deposition in hippocampus The Aβ₄₂ levels also correlated with % Aβ deposition but not as well as Aβ₄₀ levels.

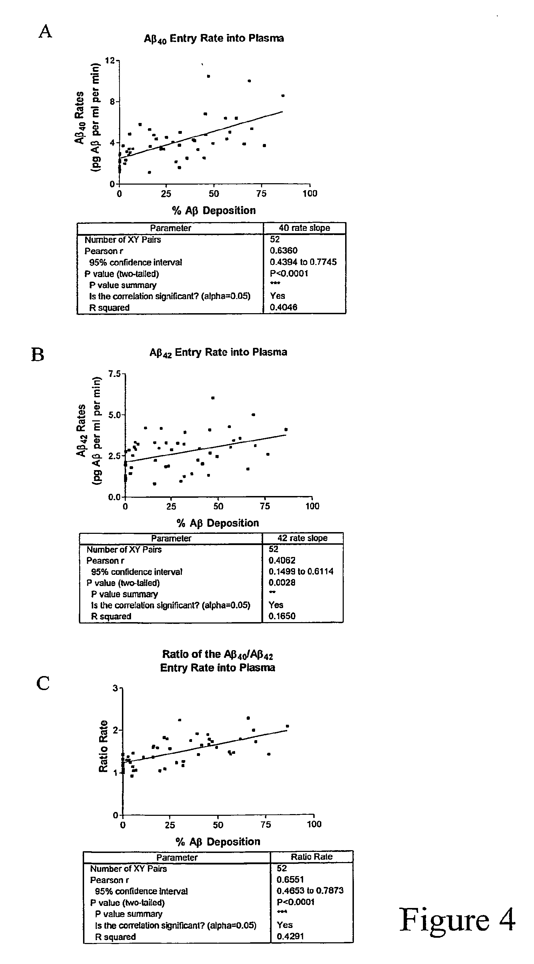

Figures 4 A, B and C show analogous results with respect to entry rate of the two Aβ peptides into the plasma and the calculated values for the entry rate as a function of the ratio of these peptides. The best correlations with Aβ deposition were rate of Aβ₄₀ entry and the ratio of Aβ₄₀/Aβ₄₂.

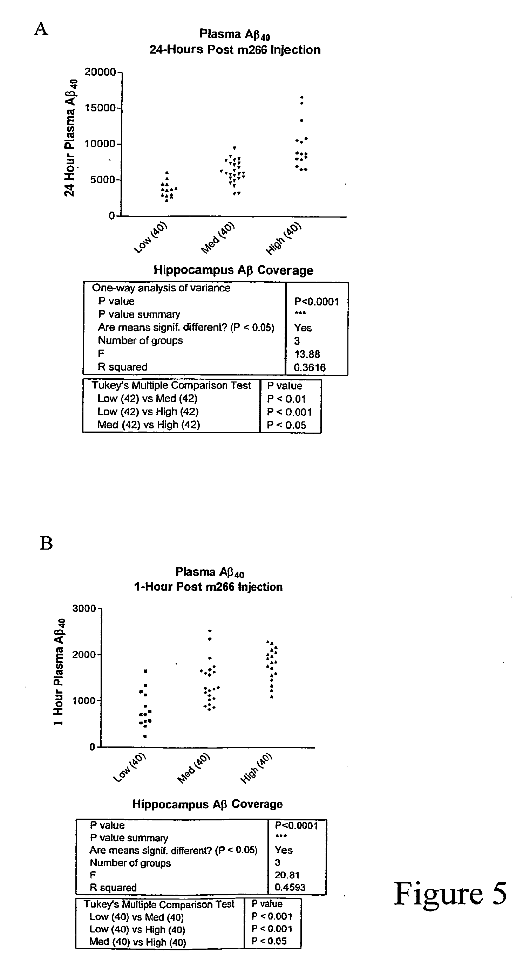

Figures 5 A and B show an alternate presentation of the data for plasma levels of Aβ₄₀ 24 hours and 1 hour after m266 injection. When the mice were grouped according to low, medium, or high Aβ coverage in the hippocampus, the animals with low Aβ deposition could be completely distinguished from those with high deposition as a function of the level of plasma Aβ₄₀.

### Example 2

In a study similar to that set forth in Example 1, a cohort of 49 homozygous APP V717F mice were used. Before and after injection of 500 µg IV of m266, plasma samples were obtained at 5 minutes, 1 hour, 3 hours, 6 hours and 24 hours and levels of Aβ₄₀ and Aβ₄₂ were assessed as described in Example 1. The mice were sacrificed after 24 hours and 1 hemisphere was assessed for the percentage of the area of the hippocampus or cingulate cortex occupied by Aβ peptide (using quantitative Aβ immunofluorescence staining) and the area occupied by amyloid (by thioflavine-S (amyloid) staining). The regions from the other hemisphere were assessed for Aβ peptide by ELISA.

The Pearson correlation coefficient (Pearson r) and significance (P value) were determined between plasma Aβ values (pre and post injection of m266) and hippocampal Aβ or amyloid load using GraphPad Prism software (version 3.00 for Windows, San Diego, USA). Aβ load is defined as the percentage area of the hippocampus covered by Aβ-immunoreactive deposits. Amyloid load is defined as the percentage area of the hippocampus covered by thioflavine-S positive deposits. Correlations were also determined between the plasma Aβ accumulation over 24 hours (area under curve, AUC) and hippocampal Aβ load or amyloid load.

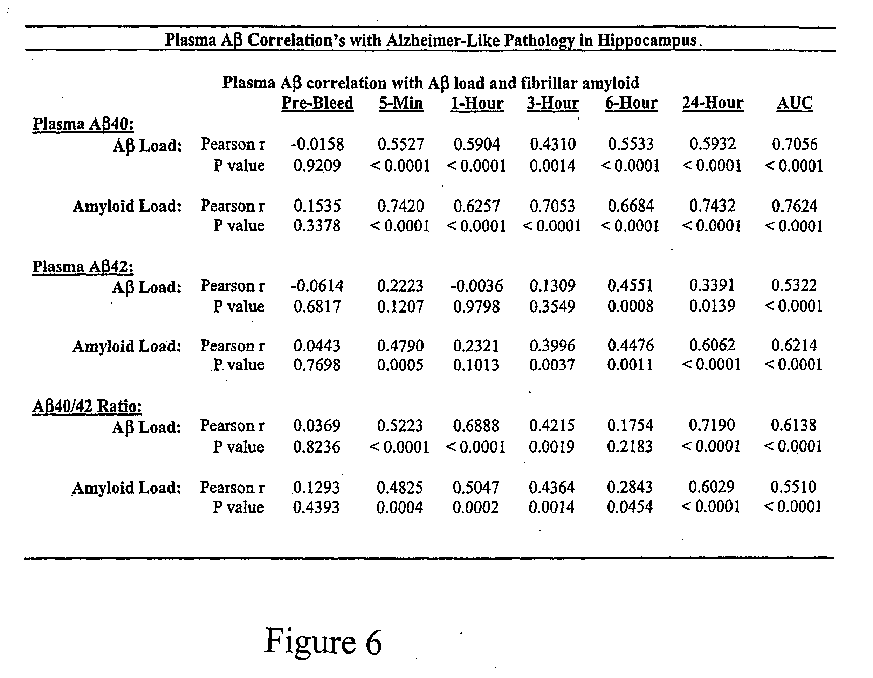

Figure 6 shown the results obtained. Briefly, it was found that the base line levels (prior to injection) of Aβ₄₀, Aβ₄₂ and the calculated Aβ₄₀/₄₂ ratio prior to injection with m266 did not correlate with percentage Aβ or amyloid deposition. However, following administration of m266, there were significant correlations between plasma Aβ₄₀, Aβ₄₂, and Aβ_{40/42} ratio with both Aβ and amyloid burden in the hippocampus and cingulate cortex.

Statistical analysis of the results permits accurate prediction of hippocampal Aβ load in these mice based on plasma Aβ₄₀ levels 24 hours following m266 injection.

## Claims

1. A method of diagnosing preclinical or clinical Alzheimer's disease comprising:
(a) measuring one or more of
(i) the level of Aβ₄₀;
(ii) the level of Aβ₄₂; or
(ii) the ratio Aβ₄₀/Aβ₄₂;
in a blood sample of a subject obtained at a time interval after administering to said subject an amount of an antibody which specifically binds an epitope contained within positions 13-28 of Aβ or an antibody that sequesters Aβ peptide from its bound, circulating form in the blood and alters clearance of soluble and bound forms of Aβ in the central nervous system in plasma, wherein said amount is effective to alter the levels of circulating Aβ peptides in the blood of said subject when said subject is in a preclinical or clinical stage of Alzheimer's disease; and
(b) comparing the level of Aβ₄₀, Aβ₄₂, or the ratio of Aβ₄₀/Aβ₄₂ in said subject with a control value of said levels, wherein elevated levels of Aβ₄₀, Aβ₄₂, or the ratio of Aβ₄₀/Aβ₄₂ in said subject as compared to control levels or ratio identifies said subject as in a preclinical or clinical stage of Alzheimer's disease.

2. The method of claim 1, wherein said time interval is less than 1 week.

3. The method of claim 1, wherein said time interval is less than or equal to 24 hours.

4. The method of claim 3, wherein the time interval is less than or equal to 3 hours.

5. The method of claim 1, wherein said administering was by injection of said antibodies.

6. The method of claim 1, wherein the subject is human and the antibody is a humanized antibody or a fragment thereof.

7. The method of claim 6, wherein the humanized antibody or fragment thereof comprises a light chain of SEQ ID NO:11 and a heavy chain SEQ ID NO:12.

8. The method of claim 6, wherein the humanized antibody or fragment thereof comprises a light chain of SEQ ID NO: 11 and a heavy chain of SEQ ID NO:16.

9. The method of claim 6, wherein the humanized antibody or fragment thereof comprises a light chain comprising a variable region of SEQ ID NO:7 and a heavy chain comprising a variable region of SEQ ID NO:14.

10. The method of claim 1, wherein said antibody is a fragment.

11. The method of claim 1, wherein the antibody is a single-chain antibody.

## Patentansprüche

1. Verfahren zur Diagnose von präklinischer oder klinischer Alzheimerschen Krankheit, umfassend:
(a) Messen von entweder
(i) dem Gehalt an Aβ₄₀;
(ii) dem Gehalt an Aβ₄₂; oder
(II) dem Verhältnis von Aβ₄₀/Aβ₄₂
oder von mehreren davon;
in einer Blutprobe eines Subjekts, die in einem Zeitintervall nach Verabreichung einer Menge an einem Antikörper an dieses Subjekt erhalten wurde, wobei der Antikörper ein innerhalb den Positionen 13-28 von Aβ enthaltenes Epitop spezifisch bindet oder der Antikörper ein Aß-Peptid aus seiner gebundenen zirkulierenden Form im Blut sequestriert und die Clearance von löslichen und gebundenen Formen von Aβ im zentralen Nervensystem in Plasma verändert, wobei die Menge effektiv ist, die Gehalte an zirkulierenden Aß-Peptiden im Blut des Subjekts zu verändern, wenn das Subjekt sich in einem präklinischen oder klinischen Stadium der Alzheimerschen Krankheit befindet; und
(b) Vergleichen des Gehalts an Aβ₄₀, Aβ₄₂ oder des Verhältnisses von Aβ₄₀/Aβ₄₂ im Subjekt mit einem Kontrollwert der Gehalte, wobei Gehalte von Aβ₄₀, Aβ₄₂ oder des Verhältnisses von Aβ₄₀/Aβ₄₂ im Objekt, die im Vergleich zu den Kontrollgehalten oder dem Kontrollverhältnis erhöht sind, das Subjekt als eines identifizieren, das sich in einem präklinischen oder klinischen Stadium der Alzheimerschen Krankheit befindet.

2. Verfahren nach Anspruch 1, wobei das Zeitintervall weniger als 1 Woche beträgt.

3. Verfahren nach Anspruch 1, wobei, das Zeitintervall weniger als 24 Stunden oder gleich 24 Stunden beträgt.

4. Verfahren nach Anspruch 3, wobei das Zeitintervall weniger als 3 Stunden oder gleich 3 Stunden beträgt.

5. Verfahren nach Anspruch 1, wobei die Verabreichung mittels Injektion der Antikörper erfolgte.

6. Verfahren nach Anspruch 1, wobei das Subjekt menschlich ist und der Antikörper ein humanisierter Antikörper oder ein Fragment davon ist.

7. Verfahren nach Anspruch 6, wobei der humanisierte Antikörper oder das Fragment davon eine leichte Kette von SEQ ID Nr. 11 und eine schwere Kette von SEQ ID Nr. 12 umfasst.

8. Verfahren nach Anspruch 6, wobei der humanisierte Antikörper oder das Fragment davon eine leichte Kette von SEQ ID Nr. 11 und eine schwere Kette von SEQ ID Nr. 16 umfasst.

9. Verfahren nach Anspruch 6, wobei der humanisierte Antikörper oder das Fragment davon eine leichte Kette, die eine variable Region von SEQ ID Nr. 7 aufweist, und eine schwere Kette umfasst, die eine variable Region von SEQ ID Nr. 14 aufweist.

10. Verfahren nach Anspruch 1, wobei der Antikörper ein Fragment ist.

11. Verfahren nach Anspruch 1, wobei der Antikörper ein Einzelketten-Antikörper ist.

## Revendications

1. Procédé de diagnostic de la maladie d'Alzheimer au stade pré-clinique ou clinique, comprenant :
(a) la mesure d'au moins :
(i) la concentration d'Aβ₄₀
(ii) la concentration d'Aβ₄₂ ou
(iii) le rapport Aβ₄₀/Aβ₄₂
dans un échantillon de sang d'un sujet, prélevé à un certain intervalle de temps après avoir administré audit sujet une certaine quantité d'un anticorps qui se lie spécifiquement à un épitope contenu au sein des positions 13-28 d'Aβ40 ou d'un anticorps qui séquestre le peptide Aβ de sa forme liée, circulante, dans le sang et altère la clairance plasmatique des formes soluble et liée d'Aβ dans le système nerveux central, dans lequel ladite quantité est efficace pour altérer les concentrations des peptides Aβ circulant dans le sang dudit sujet quand ledit sujet est dans un stade pré-clinique ou clinique de la maladie d'Alzheimer; et
(b) la comparaison de la concentration d'Aβ₄₀, d'Aβ₄₂ ou du rapport Aβ₄₀/Aβ₄₂ chez ledit sujet à une valeur témoin desdites concentrations, dans laquelle l'augmentation des concentrations d'Aβ₄₀, d'Aβ₄₂ ou du rapport Aβ₄₀/Aβ₄₂ chez ledit sujet par rapport aux concentrations témoins ou au rapport témoin identifie ledit sujet comme étant à un stade pré-clinique ou clinique de la maladie d'Alzheimer.

2. Procédé selon la revendication 1, dans lequel ledit intervalle de temps est inférieur à une semaine.

3. Procédé selon la revendication 1, dans lequel ledit intervalle de temps est inférieur ou égal à 24 heures.

4. Procédé selon la revendication 1, dans lequel ledit intervalle de temps est inférieur ou égal à 3 heures.

5. Procédé selon la revendication 1, dans lequel ladite administration est faite par injection desdits anticorps.

6. Procédé selon la revendication 1, dans lequel le sujet est humain et l'anticorps est un anticorps humanisé ou un fragment de celui-ci.

7. Procédé selon la revendication 6, dans lequel l'anticorps humanisé ou le fragment de celui-ci comprend une chaîne légère de SEQ ID NO:11 et une chaîne lourde de SEQ ID NO:12.

8. Procédé selon la revendication 6, dans lequel l'anticorps humanisé ou le fragment de celui-ci comprend une chaîne légère de SEQ ID NO:11 et une chaîne lourde de SEQ ID NO:16.

9. Procédé selon la revendication 6, dans lequel l'anticorps humanisé ou le fragment de celui-ci comprend une chaîne légère comprenant une région variable de SEQ ID NO:7 et une chaîne lourde comprenant une région variable de SEQ ID NO:14.

10. Procédé selon la revendication 1, dans lequel ledit anticorps est un fragment.

11. Procédé selon la revendication 1, dans lequel l'anticorps est un anticorps à chaîne unique.
